(19) Europäisches Patentamt
European Patent Office
Office européen des brevets

(11) **EP 4 382 909 A1**

(12) **EUROPEAN PATENT APPLICATION**

(43) Date of publication:
**12.06.2024 Bulletin 2024/24**

(21) Application number: **22212088.3**

(22) Date of filing: **07.12.2022**

(51) International Patent Classification (IPC):
**G01N 33/533** (2006.01)  **G01N 21/64** (2006.01)
**G01N 33/50** (2006.01)  **G01N 33/58** (2006.01)

(52) Cooperative Patent Classification (CPC):
**G01N 33/533; G01N 33/5005; G01N 33/582;**
G01N 21/6458; G01N 33/5008; G01N 33/5082;
G01N 2021/6421; G01N 2021/6441; G01N 2570/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Genoskin**
**31100 Toulouse (FR)**

(72) Inventor: **The designation of the inventor has not yet been filed**

(74) Representative: **Barbot, Willy**
**Simodoro-ip**
**82, rue Sylvabelle**
**13006 Marseille (FR)**

(54) **MULTIPLEX ANNOTED TISSUE IMAGING SYSTEM**

(57) The present invention concerns a new method, called Multiplexed Annotated Tissue Imaging System (MANTIS), which is directed to the analyzing of a biological sample by the steps of:

i) incubating the biological sample simultaneously with a first set of probes, said set comprising between 5 and N distinct probes, each distinct probe being specific of a distinct target, and each probe being associated with a distinct fluorophore;

ii) determining the signal, by a laser microscope and in the biological sample, for each bound probe associated with a distinct fluorophore;

iii) performing a cell segmentation of the biological sample;

iv) combining the signals obtained in step ii) for all the distinct probes for defining the expression of all the corresponding distinct targets in the cells of the biological sample as defined in step iii); and

v) characterizing the cells from the biological sample on the basis of the expression profile defined at step iv);

wherein:

o the laser microscope used for the imaging step ii) has at least n detectors with n being equal or greater then 4, preferably equal or greater than 5;

o N is equal to $2^n$; preferably to $2^{n-1}$; and

o The emission spectrum of each selected fluorophore has an overlap of less than 30% with the emission spectrum of any of the other used fluorophores.

EP 4 382 909 A1

## Description

### Technical field

Disclosed herein are systems and methods for imaging biological samples.

### Background

Various methods may be used in biology to detect and observe different molecular targets in a biological sample. Such methods are of great value in studying, diagnosing, and guiding the treatment of, a wide range of diseases. Among these methods, the vast majority of clinical diagnostics relies on immunohistochemistry (IHC) used to detect molecular targets of interest in cells and tissue, using antibodies to bind with specific targets of interest, and a labeling system that enables detection of the antibodies.

Many of the current techniques may detect only a few targets at one time (such as, IHC or fluorescence-based western blots where number of targets detectable is limited by the fluorescence-based detection system) in a single sample. Further analysis of targets may require use of additional biological samples from the source limiting the ability to determine relative characteristics of the targets such as the presence, absence, concentration, and/or the spatial distribution of multiple biological targets in the biological sample. Moreover, in certain instances, a limited amount of sample may be available for analysis, or the individual sample may require further analysis. Thus, methods, agents, and devices capable of iteratively analyzing an individual sample are needed.

Recently, highly multiplexed imaging systems have been developed. These systems have significantly advanced our understanding of cell type spatial distribution with regards to tissue structures, with a strong focus on cancer samples and tumor heterogeneity. While multiplexed imaging has an immense potential, there is a strong need to democratize these methods with the use of inexpensive instrumentation compatible with standard tissue processing and coupled to analysis user-friendly enough to be used in routine.

### Summary of the invention

The inventors developed a new method, called Multiplexed Annotated Tissue Imaging System (MANTIS), which is specifically designed for spatially-resolved phenotyping of experimental or clinical samples using routine practice and materials. Given its accuracy and flexibility, MANTIS is designed to solve the cell type spatial organization to better apprehend the cellular tissue structure.

Accordingly, a first object of the invention is directed to a method for analyzing a biological sample comprising the steps of:

i) incubating the biological sample simultaneously with a first set of probes, said set comprising between 5 and N distinct probes, preferably between 6 and N, and most preferably between 7 and N distinct probes, each distinct probe being specific of a distinct target, and each probe being associated with a distinct fluorophore;
ii) determining the signal, by a laser microscope and in the biological sample, for each bound probe associated with a distinct fluorophore;
iii) performing a cell segmentation of the biological sample;
iv) combining the signals obtained in step ii) for all the distinct probes for defining the expression of all the corresponding distinct targets in the cells of the biological sample as defined in step iii); and
v) characterizing the cells from the biological sample on the basis of the expression profile defined at step iv);

wherein:

∘ the laser microscope used for the imaging step ii) has at least n detectors with n being equal or greater then 4, preferably equal or greater than 5;
∘ N is equal to $2^n$; preferably to $2^{n-1}$; and
∘ The emission spectrum of each selected fluorophore has an overlap of less than 30% with the emission spectrum of any of the other used fluorophores, preferably less than 30% with the emission spectrum of all the other used fluorophores.

### Detailed description

As used herein, a biological sample include fresh tissues such as biopsy sections, fresh-frozen (FF) tissue sections, and formalin-fixed, paraffin-embedded (FFPE) tissue sections.

Advantageously, the biological sample is a tissue section or tissue microarray, preferably placed on a substrate like a microscope slide.

The biological sample may correspond to healthy or pathological tissue, like tissue from tumors or from inflammatory diseases.

The biological sample may be of any origin, including kidney, brain, heart, liver, pancreas, skin, intestine, testicle, etc.

Preferably, the biological sample is a skin sample.

The used laser microscope enables the obtaining of 3D images.

Preferably, the laser microscope is a confocal microscope.

The laser microscope may include multiple light sources, some of them can be LED instead of laser sources.

light source may be a LED or a laser, preferably the light source a laser.

The detector is dedicated to the detection of the light intensity from the biological sample and is transforming into an electrical signal. As typical detector, one can cite photomultiplier tube (PMT) or avalanche photodiode.

Each detector is associated to a specific detection window corresponding to a specific wavelengths window (e.g. 30 nm between 603 and 633 nm).

Thus, the emission spectrum of each fluorophore is measured by one detector of the laser microscope. Accordingly, the emission spectrum of each distinct fluorophore corresponds to the emission spectrum as measured by one detector of the laser microscope. Said emission spectrum is thus the emission spectrum of the fluorophore within the specific wavelength window of this detector.

As used herein, a fluorophore is a fluorescent chemical compound that can re-emit light upon light excitation. Fluorophores are well known from the skilled person and include xanthene, cyanine, squaraine, naphthalene, coumarin, oxadiazole, anthracene, pyrene, oxazine, acridine, arylmethine, tetrapyrrole, and dipyrromethene based dyes. Commercial fluorophores include CF dye (BIOTIUM), DRAQ and CyTRAK probes (BIOSTATUS), BODIPY (INVITROGEN), EVERFLUOR (SETAREH BIOTECH), ALEXA Fluor (INVITROGEN), BELLA Fluor (SETAREH BIOTECH), DYLIGHT Fluor (THERMO SCIENTIFIC, PIERCE), ATTO AND TRACY (SIGMA ALDRICH), FLUOPROBES (INTERCHIM), ABBERIOR Dyes (ABBERIOR), DY and MEGASTOKES Dyes (DYOMICS), SULFO CY dyes (CYANDYE), HILYTE Fluor (ANASPEC), SETA, SETAU and SQUARE Dyes (SETA BIOMEDICALS), QUASAR and CAL FLUOR dyes (BIOSEARCH TECHNOLOGIES), SURELIGHT Dyes (APC, RPEPERCP, PHYCOBILISOMES) (COLUMBIA BIOSCIENCES), APC, APCXL, RPE, BPE (PHYCO-BIOTECH, GREENSEA, PROZYME, FLOGEN), and VIO Dyes (MILTENYI BIOTEC).

Preferably, the fluorophore is selected among AF532, AF488, AF546, AF594, AF647, AF700, APC-CY7, BV650, EFLUOR 660, EFLUOR 450, AND SB645.

Preferably, the fluorophore are selected among:

1) AF-532, AF488, AF594, APC-Cy7, BV650, eFluor 660, and eFluor 450

or

2) AF-532, *AF405,* AF488, AF647, AF546, *AF594,* AF700, and SB645.

The fluorophore associated with the probe may be linked or not to the probe.

In fact, such association may be done using a secondary antibody liked to the fluorophore, whose secondary antibody is directed against the primary antibody corresponding to the probe.

Preferably, each distinct probe is linked to its distinct fluorophore.

As used herein, a probe refers to any compound directed against a specific target. Accordingly, a probe may correspond to an oligonucleotide whose sequence is complementary to the one of a target sequence or to an antibody or an antibody fragment targeting a specific target.

Preferably, the probe is an antibody or an antibody fragment.

The antibody or antibody fragment can include any one of different types of antibody species, including but not limited to, an immunoglobulin G (IgG), an immunoglobulin M (IgM), a polyclonal antibody, a monoclonal antibody, a single-chain fragment variable (scFv) antibody, a nanobody, an antigen-binding fragment (Fab), and a diabody. Antibodies and antibody fragments can be of mouse, rat, rabbit, human, camelid, or goat origin. In some embodiments, the antibody or antibody fragment can be raised against a human, mouse, rat, cow, pig, sheep, monkey, rabbit, fruit fly, frog, nematode or woodchuck antigen. In certain embodiments, the antibody or antibody fragment can be raised against an animal, plant, bacteria, fungus, or protist antigen.

Targets are well known from the skilled person and may be any marker useful for characterizing, and thus phenotyping, any cell.

The step iii) of performing cell segmentation of the biological sample is done by delimiting the single cells as object in the biological sample. Such step can be done using well known software such as the IMARIS software (BITPLANE). Such cell segmentation step is done on the basis of the signal of a probe directed against a general cellular marker (e.g. CD45 for immune cells), general membrane or nuclear markers (e.g. signal resulting from DAPI staining).

The step iv) is realized by methods -i.e. softwares- well known from the skilled person. Advantageously, said step iii) include a spectral unmixing and deconvolution step using softwares well known from the skilled person and depending on the laser microscopes. As an examples of such softwares, one can cite softwares HUYGENS.

The step v) of characterizing cells from the biological sample on the basis of the expression profile defined at step iv) is done by comparing the expression of all the corresponding distinct targets in each cell of the biological sample as defined at step iii) with defined biomarkers' signatures.

This step is preferably based on phenotype attribution matrices identifying correlations between the expression profiles of different biomarkers.

For such correlation determination, many well known methods can be used. As an example, a correlation can be performed by a pairwise Spearman's Rank Correlation (

$$\rho = 1 - \frac{6 \sum d_i^2}{n(n^2 - 1)}$$

) using R software.

Practically, a table containing defined biomarkers' signatures was built and compared with each identified cell.

For each cell, the value is set to 1 when the detected markers are the ones of the defined biomarkers' signatures, otherwise it is set to 0. In this case, the corresponding table is disclosed below.

Alternatively, the set value can correspond to the MFI value obtained for the analyzed cell, and a pairwise Spearman's correlation analysis was then done for each detected single-cell, against all possible combinations of biomarkers to identify the most relevant biomarkers' signature. The output information is the attribution of specific rho values per single-cell which then automatically finds the best match of cellular identity and generates associated quantitative statistics. Thus, each cell is phenotypically assigned to the biomarkers' signature having the highest correlation coefficient, whereas cells with multiple highest correlation coefficients were assigned as "Other" cell types.

According to a first preferred embodiment, the method of the invention is for identifying and characterizing immune cells among the biological sample, wherein each probe of the first set is directed against immune cells markers and, eventually, immune cells activation markers.

Immune cells markers are well known from the skilled person and includes AHR, Bc1-6, BCMA, CD1c, CD3, CD4, CD8, CD11b, CD11c, CD14, CD15/SSEA1, CD16, CD19, CD20, CD25, CD33, CD45, CD56, CD57, CD66b, CD68, CD117, CD123, CD138, CD163, CD172a, CD193, CD207, CD209, CLEC9A, CTLA4, CXCR5, Epcam, FcER1a, FoxP3, GATA-3, HLA-DR, IFN gamma, IL-4, IL-9, IL-17, IL-21, IL-22, myeloperoxidase (MPO), NCR, PU.1, RORgt, Siglec8, SIRPa, T-Bet, TCR Va24Vb11, TCR gamma/delta, Tryptase, and XCR1.

Advantageously, the first set of probes comprises at least one probe directed against CD45.

Activation markers from immune cells are also well known from the skilled person and includes CCR7, CD80, CD83, CD86, CD40, pan-HLA, CD57, Ki67, Caspase 3 active, PDL1, CTLA4, c-kit, CD203c, CD63, CD69, Granzyme, Perforine, CD62L, TCF1/TCF7, PD-1, TIM-3, ToxITox2, and TIGIT.

The defined biomarkers' signatures for immune cells can be identified by the skilled person in view of his general knowledge.

Preferably, the defined biomarkers' signatures of step v) are as follows:

- Langerhans cells: CD45, CD11b, CD207, Epcam;

- Dermal dendritic cells (cDC1): CD45, CD11b, CD11c, XCR1, CLEC9A

- Dermal dendritic cells (cDC2): CD45, CD11b, CD1c, CD11c, SIRPa, CD172a

- Macrophages: CD45, CD11b, CD68, CD163

- Mast cells: CD45, CD11b, Siglec8, Tryptase, FcER1a, CD117, CD33

- Eosinophils: CD45, CD11b, Siglec 8, CD193

- Basophils: CD45, CD11b, Cd123, Siglec 8, FcERIa

- Neutrophils: CD45, CD11b, MPO, CD16, CD66b, CD15/SSEA1

- Monocytes: CD45, CD11b, CD14, CD209

- B cells: CD45, CD20, CD19

- NK cells: CD45, CD56, TCR Va24Vb11, NCR

- NK T cells: CD45, CD4, CD56, TCR Va24Vb11

- Helper T cells: CD45, CD3e, CD4, PU.1, IL-9, T-Bet, IFNg, GATA-3, IL-4, Bcl-6, CXCR5, IL-21, AHR, IL-22, RORgt, IL-17, FoxP3, CD25, CTLA4

- Cytotoxic T cells: CD45, CD3e, CD8a

- Tgd cells: CD45, CD3e, TCR gamma/delta; and

- Plasma cells: CD45, BCMA, CD138

Preferably, the first set of probes consists in probes directed against the markers CD1c, CD3, CD4, CD8, CD20, CD45, CD57, CD123, CD207, HLA-DR, MPO, Siglec8, TCR gamma/delta, and/or Tryptase.

Thus, the corresponding defined biomarkers' signatures of step v) are as follows:

| Immune cell population | Biomarkers' signature |
|---|---|
| B cells | CD45$^+$ CD20$^+$ |
| NK cells | CD45$^+$ CD20$^-$ CD3$^-$ CD57$^+$ |
| CD4$^+$ T cells | CD45$^+$ CD20$^-$ CD3$^+$ TCRgd$^-$ CD4$^+$ CD8$^-$ CD57$^{low\ or\ high}$ |
| CD8$^+$ T cells | CD45$^+$ CD20$^-$ CD3$^+$ TCRgd$^-$ CD4$^-$ CD8$^+$ CD57$^{low\ or\ high}$ |
| gd T cells | CD45$^+$ CD20$^-$ CD3$^+$ TCRgd$^+$ |
| dn T cells | CD45$^+$ CD20$^-$ CD3$^+$ TCRgd$^-$ CD4$^-$ CD8$^-$ |
| dp T cells | CD45$^+$ CD20$^-$ CD3$^+$ TCRgd$^-$ CD4$^+$ CD8$^+$ |
| Mast cells | CD45$^+$ Tryptase$^+$ |
| DC | CD45$^+$ CD1c$^+$ CD207$^-$ HLA-DR$^{low\ or\ high}$ |
| LC | CD45$^+$ CD1c$^-$ CD207$^+$ HLA-DR$^{low\ or\ high}$ |
| DC CD207$^+$ | CD45$^+$ CD1c$^+$ CD207$^+$ HLA-DR$^{low\ or\ high}$ |
| Neutrophils | CD45$^+$ CD1c$^-$ CD207$^-$ Tryptase- Siglec8$^-$ MPO$^+$ |
| Eosinophils | CD45$^+$ CD1c$^-$ CD207$^-$ MPO$^-$ Tryptase$^-$ Siglec8$^+$ CD123$^-$ |
| Basophils | CD45$^+$ CD1c$^-$ CD207$^-$ MPO$^-$ Siglec8$^+$ CD123$^+$ |

Now and advantageously, the immune cells are lymphoid cells and the first set of probes consists in probes directed against the markers CD3, CD4, CD8, CD20, CD45, CD57, and/or TCR gamma/delta.

Thus, the corresponding defined biomarkers' signatures of step v) are as follows:

| Immune cell population | Biomarkers' signature |
|---|---|
| B cells | CD45$^+$ CD20$^+$ |
| NK cells | CD45$^+$ CD20$^-$ CD3$^-$ CD57$^+$ |
| CD4$^+$ T cells | CD45$^+$ CD20$^-$ CD3$^+$ TCRgd$^-$ CD4$^+$ CD8$^-$ CD57$^{low\ or\ high}$ |
| CD8$^+$ T cells | CD45$^+$ CD20$^-$ CD3$^+$ TCRgd$^-$ CD4$^-$ CD8$^+$ CD57$^{low\ or\ high}$ |

(continued)

| Immune cell population | Biomarkers' signature |
|---|---|
| gd T cells | CD45$^+$ CD20$^-$ CD3$^+$ TCRgd$^+$ |
| dn T cells | CD45$^+$ CD20$^-$ CD3$^+$ TCRgd$^-$ CD4$^-$ CD8$^-$ |
| dp T cells | CD45$^+$ CD20$^-$ CD3$^+$ TCRgd$^-$ CD4$^+$ CD8$^+$ |

Still advantageously but alternatively, the immune cells are myeloid cells and the first set of probes consists in probes directed against the markers CD1c, CD45, CD57, CD123, HLA-DR, MPO, Siglec8, and/or Tryptase.

Thus, the corresponding defined biomarkers' signatures of step v) are as follows:

| Immune cell population | Biomarkers' signature |
|---|---|
| Mast cells | CD45$^+$ Tryptase$^+$ |
| DC | CD45$^+$ CD1c$^+$ CD207$^-$ HLA-DR$^{low\ or\ high}$ |
| LC | CD45$^+$ CD1c$^-$ CD207$^+$ HLA-DR$^{low\ or\ high}$ |
| DC CD207$^+$ | CD45$^+$ CD1c$^+$ CD207$^+$ HLA-DR$^{low\ or\ high}$ |
| Neutrophils | CD45$^+$ CD1c$^-$ CD207$^-$ Tryptase$^-$ Siglec8$^-$ MPO$^+$ |
| Eosinophils | CD45$^+$ CD1c$^-$ CD207$^-$ MPO$^-$ Tryptase$^-$ Siglec8$^+$ CD123$^-$ |
| Basophils | CD45$^+$ CD1c$^-$ CD207$^-$ MPO$^-$ Siglec8$^+$ CD123$^+$ |

According to another preferred embodiment, the method of the invention is for identifying and characterizing structural cells among the biological sample, wherein each probe of the first set is directed against structural cells markers.

Structural cells are well known from the skilled person and includes ACTA2, ADIPOQ, ADIPOR1, ADIPOR2, APCDD1, ASPN, AXIN2, B3TUB, CALCA, CAPN3, CCL19, CDC42EP3, CIDEA, CITED1, COL1A1, COL6A1, COL6A2, COL3A1, COLEC12, COL18A1, CTHRC1, DCN, DCT, FABP4, FGF21, GLDN, GLUT4/SLC2A4, HRT15, IGFBP7, KRT14, KRT5, KRT1, KRT10, KRT6A, KRT16, KRT6b, KRT17, KRT79, LUM, MFAP4, MGP, MFAP5, MLANA, MITF , MMP2, MRGPRA, MRGPRD, NRXN1, PECAM1, PGFRA, PDGFRA PHLDA1, PGP9.5, PLIN1, PLIN2, PNPLA2, PMEL, PTGDS, QPCT, TAC1, TAGLN, TOP2A, TRPV1, TYR, TYRP1, UBEC2, UCP1, VIM, and VWF.

Thus, the corresponding defined biomarkers' signatures of step v) are as follows:

- Keratinocytes: HRT15, KRT14, KRT5, KRT1, KRT10, KRT6A, KRT16, TOP2A, UBEC2, KRT6b, KRT17

- Melanocytes: MLANA, PMEL, TYRP1, DCT, CDC42EP3, MITF, QPCT, IGFBP7, TYR, CAPN3, PHLDA1

- Fibroblasts: COL1A1, DCN, COL6A1, COL6A2, MFAP4, COL3A1, MMP2, LUM, VIM, PDGFRA, APCDD1, AXIN2, COLEC12, PTGDS, COL18A1, MGP, MFAP5, CTHRC1, APCDD1, CCL19, ASPN, PGFRA

- Sebaceous gland cells: KRT79

- Pericytes: TAGLN, ACTA2

- Neuronal cells: NRXN1, TRPV1, MRGPRD, MRGPRA, TAC1, CALCA, B3TUB, PGP9.5

- Schawn cells: GLDN

- VEC: PECAM1, VWF

- Adipocytes: FABP4, PLIN1, PNPLA2, UCP1, CIDEA, CITED1, FGF21, ADIPOR1, ADIPOR2, GLUT4/SLC2A4, PLIN2, ADIPOQ.

The method of the invention can further include, preferably after the step ii), a step of applying a stain to the biological sample.

The stain can include at least one member selected from the group consisting of a counterstain, a chromogenic stain, and an immunofluorescent stain. Preferably, the stain is a histochemical dye such as eosin, hematoxylin, 4',6-diamidino-2-phenylindole (DAPI), periodic acid-Schiff stain (PAS), methylene blue, and HOECHST stains (such as HOECHST 33342, HOECHST 34580, HOECHST 333258). Such a step can provide information about the presence, location, and state of various cellular compartments in the biological sample.

Eventually, the method of the invention can undergo several steps i) and ii) by incubating the biological sample simultaneously with a second or further set of probes, said second or further set comprising between 3 and N distinct probes, each distinct probe being as defined previously; and ii) determining the signal, by a laser microscope and in the biological sample, for each probe of this new set.

Now, the method of the invention preferably comprises only a single round of incubating step i) of a biological sample with a set of probes.

If the step ii) can be interrupted by storage of the biological sample at low temperature (e.g. approximately 4°C), the signal determination is preferably done consecutively for all the distinct fluorophores without any storage.

The method of the invention may comprise, before the step ii), a step of treating the biological sample with an autofluorescence quenching solution. Such solutions are well known from the skilled person and include, as an example, the autofluorescence quenching solution TRUE VIEW (VECTOR LAB).

In the following, the invention is described in more detail. Yet, no limitation of the invention is intended by the details of the examples. Rather, the invention pertains to any embodiment which comprises details which are not explicitly mentioned in the examples herein, but which the skilled person finds without undue effort.

**Examples**

1) Multiplex imaging initial strategy

Human skin samples were either frozen in optimal cutting temperature compounds (OCT, TISSUE-TEK) or formalin-fixed and paraffin embedded (FFPE).

10 $\mu$m FFPE-tissue sections were heated at 95°C for 20 minutes. Sections were subsequently sunk into Xylene for 30 minutes, washed in 100% ethanol, 95% ethanol, 70% ethanol, 50% ethanol, 30% ethanol for 5 minutes each and abundantly washed with distilled water. They were then treated using a heat-induced epitope retrieval method as previously described (Serhan et al., 2019). FFPE-tissue sections were blocked and permeabilized with PBS 0.5 % (w/v)% BSA (SIGMA-ALDRICH), 0.3 % TRITON X-100 (MERCK) for 30 minutes to 1 hour at room temperature, then incubated with fluorophore-coupled antibodies or unconjugated antibodies overnight at 4°C in the dark.

Compared to classical segmentation strategies based on nucleus expansion, which can often lead to under or overestimations of cellular clusters composition, the general immune biomarker CD45 was used as a robust immune staining visualized in most skin-resident immune cells to constitute the core of our cell segmentation strategy for future single immune cell statistics extraction. All conjugated and unconjugated antibodies used in this study were validated in single stainings of human skin and amygdala and are listed in the following Table.

| Antibody | Clone | Fluorochrome | Concent. | Reference |
|---|---|---|---|---|
| CD45 | HI30 | AF-532 | 0.12 $\mu$g | THERMOFISHER, #58-0459-42 |
| CD4 | N1UG0 | Unconjugated *Revealed with anti-mouse AF488* | 10 $\mu$g/ml | EBIOSCIENCE, #14-2444-82 |
| CD3 | Polyclonal | Unconjugated *Revealed with anti-rabbit AF594* | 10 $\mu$g/ml | AGILENT, #A045229-2 |
| CD20 | 2H7 | APC-Cy7 | 20 $\mu$g/ml | BIOLEGEND, #302313 |
| TCR-gd | B1 | BV650 | 15 $\mu$g/ml | BD, #564156 |
| CD8a | AMC908 | eFluor 660 | 10 $\mu$g/ml | THERMOFISHER #50-0008-80 |
| CD57 | TB01 | eFluor 450 | 10 $\mu$g/ml | THERMOFISHER, #48-0577-41 |

The tissue sections were then washed three times in PBS 0.5 % (w/v)% BSA, 0.3 % TRITON X-100 and incubated, if needed, with secondary antibodies in PBS 0.5 % (w/v)% BSA, 0.3 % TRITON X-100 for 2 hours in the dark. Finally, samples were treated with an autofluorescence quenching solution named TRUE VIEW (VECTOR LAB) for 5 minutes. Slides were mounted in MOWIOL medium (SIGMA-ALDRICH) and sealed with a coverslip.

The 3D fluorescent multiplexed images were then acquired using an 8-year-old confocal microscope SP8 (LEICA MICROSYSTEMS) equipped with a HC PL APO CS2 with 40X NA 1.3 oil objective, a UV diode (405nm) and four lasers in visible range wavelengths (405nm, 488nm, 532nm, 552nm and 635nm). The used detection windows and microscope configuration are listed in the following table.

| Antibody | Fluorochrom e | Excitation laser (nm) | Detection window |
|----------|---------------|----------------------|------------------|
| CD45 | AF-532 | 532 | 535-578 nm |
| CD4 | AF488 | 488 | 503-539 nm |
| CD3 | AF594 | 552 | 603-633 nm |
| CD20 | APC-Cy7 | 635 | 740-790 nm |
| TCR-gd | BV650 | 405 | 624-682 nm |
| CD8a | eFluor 660 | 635 | 651-693 nm |
| CD57 | eFluor 450 | 405 | 415-479 nm |

The obtained 3-D images were then deconvoluted and compensated to correct 3D fluorescent spectral spillovers using the HUYGENS software (SCIENTIFIC VOLUME IMAGING), a strategy routinely applied in flow cytometry to combine multiple fluorochromes simultaneously.

The 3D fluorescence signal of CD45 for individual immune cells was then modeled using the Isosurface algorithm of the IMARIS software (BITPLANE) and exported a corresponding single-cell database composed of the mean fluorescence intensity (MFI) of all individual biomarkers and precise x,y,z tissue coordinates obtained with a resolution of 250 nm/pixel.

The inventors established that CD45-based segmentation enabled an efficient isolation of single immune cells characteristics, even when those were found aggregated around dermal structural elements. Overall, the inventors demonstrate that it is possible to extract a 10 parameters single-cell database using regular confocal equipment coupled to basic computational imaging steps.

2) Multiplex imaging method optimization

Then, the inventors designed two panels composed of antibodies directed against immune biomarkers suitable to generate a non-exhaustive overview of lymphoid and myeloid cells landscape of the skin. The combination of CD45, CD3e, CD4, CD8, TCRγδ, CD20 and CD57 (a terminally sulfated glycan carbohydrate epitope shared by NK and T cells with high cytotoxic potential) allows to identify the following lymphoid cells: conventional CD4 and CD8 T cells (being CD57$^{low}$ or CD57$^{high}$), γδ T cells, B cells and NK cells. The combination of CD45, CD207, CD1c, HLA-DR, CD123, Siglec-8, myeloperoxidase (MPO) and tryptase allows to identify the following myeloid cells: Langerhans cells, Langerin$^+$ dermal dendritic cells (dDCs) and Langerin$^-$ dDCs, eosinophils, basophils, neutrophils and mast cells.

The full list of antibodies is detailed in the following table.

| Antibody | Clone | Fluorochrome | Concent. | Reference |
|----------|-------|--------------|----------|-----------|
| CD45 | HI30 | AF-532 | 0.12 μg | THERMOFISH ER, #58-0459-42 |
| **Lymphoid panel** | | | | |
| CD4 | N1UG0 | Unconjugated<br>*Revealed with anti-mouse AF488* | 10 μg/ml | EBIOSCIENCE ™, #14-2444-82 |
| CD3 | Polyclona 1 | Unconjugated<br>*Revealed with anti-rabbit AF594* | 10 μg/ml | AGILENT, #A045229-2 |
| CD20 | 2H7 | APC-Cy7 | 20 μg/ml | BIOLEGEND, #302313 |
| TCR-gd | B1 | BV650 | 15 μg/ml | BD, #564156 |
| CD8a | AMC908 | eFluor 660 | 10 μg/ml | THERMOFISH ER, #50-0008-80 |

(continued)

| Lymphoid panel | | | | |
|---|---|---|---|---|
| CD57 | TB01 | eFluor 450 | 10 µg/ml | THERMOFISH ER, #48-0577-41 |
| **Myeloid panel** | | | | |
| Siglec8 | Polyclona 1 | eFluor 450<br>*Revealed with anti-rabbit AF405* | 1:50 | THERMOFISH ER, #PA5-110774 |
| CDlc | L161 | Zenon AF488 Using Zenon Mouse IgG1 labeling kit (THERMOFISHER, #Z25000) | 10 µg/ml | BIOLEGEND, #331502 |
| Tryptase | AA1 | Zenon AF647 Using Zenon Mouse IgG 1 labeling kit (THERMOFISHER, #Z25000) | 1:50000 | ABCAM, #AB2378 |
| CD207 | 923B7 | AF546 | 5µg/ml | BIOTECHNE, #DDX0373A54 6 |
| MPO | Polyclona 1 | Unconjugated<br>*Revealed with anti-goat-AF594* | 5 µg/ml | BIOTECHNE, #AF3667 |
| HLA | L243 | AF700 | 10 µg/ml | BIOTECHNE,#NB 100-77855AF700 |
| CD123 | 6H6 | SB645 | 1 µg | EBIOSCIENCE ™, #64-1239-42 |

The excitation/emission/detection strategies for all antibodies is detailed in the following table.

| Antibody | Fluorochrome | Excitation laser (nm) | Detection window |
|---|---|---|---|
| CD45 | AF-532 | 532 | 535-578 nm |
| CD4 | AF488 | 488 | 503-539 nm |
| CD3 | AF594 | 552 | 603-633 nm |
| CD20 | APC-Cy7 | 635 | 740-790 nm |
| TCR-gd | BV650 | 405 | 624-682 nm |
| CD8a | eFluor 660 | 635 | 651-693 nm |
| CD57 | eFluor 450 | 405 | 415-479 nm |
| Siglec8 | eFluor 450 | 405 | 409-470 nm |
| CDlc | AF488 | 488 | 505-536 nm |
| Tryptase | AF647 | 635 | 648-689 nm |
| CD207 | AF546 | 552 | 558-588 nm |
| MPO | AF594 | 552 | 608-633 nm |
| HLA | AF700 | 635 | 699-782 nm |
| CD 123 | SB645 | 405 | 625-681 nm |

The inventors next develop an adaptable analytical system that could integrate, and batch-process extracted single-cell databases and enable an unsupervised phenotyping of immune subsets.

To address this latter challenge, the inventors developed an interactive digital tool based on phenotype attribution matrices identifying correlations between the expression profiles of different biomarkers.

First, a table containing a literature-based theoretical signature of biomarkers expressed in each cell population identified by the used panel (naturally depending on the used set of antibodies) was built and tested with each identified cell. For each cell, the value is set to 1 when the detected markers are the ones of the corresponding and specific immune cell population, otherwise it is set to 0. In this case, the corresponding table is disclosed below.

| Immune cell population | Markers | Value (1 or 0) |
| --- | --- | --- |
| B cells | CD45$^+$ CD20$^+$ | |
| NK cells | CD45$^+$ CD20$^-$ CD3$^-$ CD57$^+$ | |
| CD4$^+$ T cells | CD45$^+$ CD20$^-$ CD3$^+$ TCRgd$^-$ CD4$^+$ CD8$^-$ CD57$^{low\ or\ high}$ | |
| CD8$^+$ T cells | CD45$^+$ CD20$^-$ CD3$^+$ TCRgd$^-$ CD4$^-$ CD8$^+$ CD57$^{low\ or\ high}$ | |
| gd T cells | CD45$^+$ CD20$^-$ CD3$^+$ TCRgd$^+$ | |
| dn T cells | CD45$^+$ CD20$^-$ CD3$^+$ TCRgd$^-$ CD4$^-$ CD8$^-$ | |
| dp T cells | CD45$^+$ CD20$^-$ CD3$^+$ TCRgd$^-$ CD4$^+$ CD8$^+$ | |
| Mast cells | CD45$^+$ Tryptase$^+$ | |
| DC | CD45$^+$ CD1c$^+$ CD207$^-$ HLA-DR$^{low\ or\ high}$ | |
| LC | CD45$^+$ CD1c$^-$ CD207$^+$ HLA-DR$^{low\ or\ high}$ | |
| DC CD207$^+$ | CD45$^+$ CD1c$^+$ CD207$^+$ HLA-DR$^{low\ or\ high}$ | |
| Neutrophils | CD45$^+$ CD1c$^-$ CD207$^-$ Tryptase$^-$ Siglec8$^-$ MPO$^+$ | |
| Eosinophils | CD45$^+$ CD1c$^-$ CD207$^-$ MPO$^-$ Tryptase$^-$ Siglec8$^+$ CD123$^-$ | |
| Basophils | CD45$^+$ CD1c$^-$ CD207$^-$ MPO$^-$ Siglec8$^+$ CD123$^+$ | |

In practice, the value "1" in the table was replaced by the maximum Mean Fluorescence Intensity (MFI) value acquired in the corresponding channel from the tested sample.

Then, a correlation matrix between the MFI table and the adapted reference panel was generated by performing a pairwise Spearman's Rank Correlation (

$$\rho = 1 - \frac{6 \sum d_i^2}{n(n^2 - 1)}$$

) using R software. In practice, the method runs instantaneously a pairwise Spearman's correlation analysis, for each detected single-cell, against all possible combinations of biomarkers to identify the immune subsets annotated in the phenotype attribution matrices. The output information is the attribution of specific rho values per single-cell which then automatically finds the best match of cellular identity and generates associated quantitative statistics. Thus, each cell is phenotypically assigned to the cell type having the highest correlation coefficient, whereas cells with multiple highest correlation coefficients were assigned as "Other" cell types.

3) acral lesion analysis

Because acral lesions are rarely biopsied when observed in COVID-19 patients, a description of pathological features is currently missing, which impairs the development of a clear readout to better diagnose and treat such skin conditions. A possible explanation could be a collateral clinical manifestation of efficient anti-viral type 1 interferon response, since acral lesions are also commonly observed in patients with interferonopathy, such as lupus erythematosus (SLE).

In a first step, the inventors generated data from two serial sections of an acral lesion from a patient with lupus erythematosus (SLE) stained with a lymphoid and a myeloid panel. The fast 3D acquisition of one region of interest (ROI) composed of 6 field of view (i.e., 0.6 mm (x) $\times$ 0.4 mm (y) $\times$ 20 $\mu$m (z)) enabled the annotation of 519 myeloid cells and 708 lymphoid cells for a total of 19 different immune subsets identified.

Then, the inventors decided to benchmark the effective performance of the method of the invention to resolve the immune topology of FFPE acral lesions of similar clinical severity from 5 COVID-Toes patients, 2 patients with a rare Kawasaki syndrome (i.e., a severe systemic inflammatory condition triggered upon Sars-cov-2 infection) and 3 patients with SLE. Abdominal skin biopsies from 5 healthy-looking controls were used to set the baseline of a natural steady state immune environment, albeit being at a distant anatomical region.

The quantitative performance of the method of the invention to annotate immune cells by calculations of statistical correlations was validated with a supervised approach of histo-cytometry applied on the same datasets for each antibody panel in all skin samples. This last method consists in a manual gating of immune subsets on the same principle used in traditional flow cytometry. A total of 20,464 single $CD45^+$ immune cells were identified with the following distribution per condition: 1,670 immune cells in 5 healthy-looking skin samples (i.e., with 895 myeloid and 775 lymphoid cells), 1,703 in 2 Kawasaki syndrome patients (i.e., with 932 myeloid and 775 lymphoid cells), 5,076 in 3 SLE patients (i.e., with 1,560 myeloid and 3,516 lymphoid cells) and 12,015 in 5 COVID-Toes patients (i.e., with 2,838 myeloid and 9,177 lymphoid cells). A classical gating strategy based on mutually exclusive biomarkers was used to assess the presence of lymphoid and myeloid cell subsets by histo-cytometry.

Accordingly, a total of 19 different immune subsets were identified and found very similar distributions of cell counts by either supervised histo-cytometry or the method of the invention, regardless of the antibody panel, the patients analyzed or the disease conditions. Importantly, all healthy-looking skin samples exhibited a proportion of immune cells aligned with previously described skin-resident immune populations at steady state in human. Now, a slightly higher tendency to detect rare populations of $CD45^+CD3^-CD20^+$ B cells or $CD45^+CD3^-CD20^+$ basophils was noticed when 3-D images were computationally analyzed with the method of the invention. This is consistent with recent studies that identified the presence of rare B cells and basophils in healthy human and mouse skin.

Compared to healthy-looking samples, acral lesions showed the presence of large immune infiltrates confirming their inflammatory status. All three pathologies were associated with an infiltration of myeloid cells composed of a large number of neutrophils, eosinophils, mast cells and conventional $CD45^+CD1c^+CD207^-HLA-DR^+$ dDCs. While detected in relatively low numbers in all analyzed skin samples, no difference was observed between healthy-looking and pathogenic samples for $CD45^+CD1c^-CD207^+HLA-DR^+$ LCs or $CD45^+CD1c^+CD207^+HLA-DR^+$ dDCs populations.

Compared to Kawasaki syndrome patients, SLE and COVID-19 patients showed a tendency to have an increased proportion of lymphoid cells, with an enrichment in conventional $CD4^+$ or $CD8^+$ T cells and NK cells, and to a lesser extent in $\gamma\delta$ T cells. Interestingly, double positive (DP) $CD45^+CD4^+CD8^+CD3^+TCR\gamma\delta^-$ and double negative (DN) $CD45^+CD4^-CD8^-CD3^+TCR\gamma\delta^-$ T cells were observed in all inflamed and some healthy-looking samples, albeit in smaller numbers. Such populations of T cells were often understudied, as CD4 and CD8 biomarkers are thought to be mutually exclusive, however they have been previously reported multiple times in autoimmune and chronic inflammatory disorders (including SLE patients).

A clustering of all patients and healthy-looking controls was then performed on the basis of the quantitative analysis of their immune signature using both a detailed heatmap per cell population and a principal component analysis (PCA) per patient. The results established that healthy-looking skin samples clustered together, without apparent relationship with the pathological samples, whereas Kawasaki syndrome and COVID-Toes patients tended to form disease-specific clusters, and SLE patients were distributed between both conditions. Albeit having a restricted number of patients, these data suggest that all analyzed acral lesions displayed common quantitative immune features, with nevertheless potential disease-intrinsic characteristics. To explore further this hypothesis, the analysis was refined by investigating the activation status of conventional $CD4^+$ and $CD8^+$ T cells based on their expression level of CD57, a biomarker classically associated with high cytotoxic potential (i.e., pro tissue damage) during viral infections and autoimmune disorders, including COVID-19. The results have shown that, compared to other pathological conditions, the 3 COVID-Toes patients were particularly enriched in $CD4^+$ and $CD8^+$ T cells exhibiting high levels of CD57 (i.e., $CD57^{high}$; calculated as CD57 MFI z-score). Moreover, and compared to Kawasaki syndrome, $CD8^+$ $CD57^{high}$ (but not $CD57^{low}$) T cells were found significantly enriched nearby the epidermis in acral lesions from COVID-Toes and SLE patients. Albeit on a limited number of patients, these findings strongly suggest the potential formation of tissue damaging subepidermal inflammatory clusters composed of cytotoxic T cells in COVID-Toes lesions.

There is a strong need to design new tools to assist clinical decision making and/or better apprehend the complexity of inflammatory dermatoses. While very promising processes have been made in the field of spatial biology, there is an unmet need for a non-expensive and standardized multiplexed imaging analytical framework capable of automatically resolving the immune architecture of an inflamed tissue. Here we show that the method of the invention is uniquely positioned to examine numerous questions in the fields of skin immuno-biology and should lay the foundation for a fast and automated analysis pipeline of in situ relevant inflammatory environments in both research and clinical facilities.

**Claims**

1. A method for analyzing a biological sample comprising the steps of:

   i) incubating the biological sample simultaneously with a first set of probes, said set comprising between 5 and N distinct probes, each distinct probe being specific of a distinct target, and each probe being associated with a distinct fluorophore;
   ii) determining the signal, by a laser microscope and in the biological sample, for each bound probe associated with a distinct fluorophore;
   iii) performing a cell segmentation of the biological sample;
   iv) combining the signals obtained in step ii) for all the distinct probes for defining the expression of all the corresponding distinct targets in the cells of the biological sample as defined in step iii); and
   v) characterizing the cells from the biological sample on the basis of the expression profile defined at step iv);

   wherein:

   ∘ the laser microscope used for the imaging step ii) has at least n detectors with n being equal or greater then 4, preferably equal or greater than 5;
   ∘ N is equal to $2^n$; preferably to $2^{n-1}$; and
   ∘ The emission spectrum of each selected fluorophore has an overlap of less than 30% with the emission spectrum of any of the other used fluorophores, preferably less than 30% with the emission spectrum of all the other used fluorophores.

2. The method of claim 1, wherein the biological sample is a tissue section or a tissue microarray, preferably the biological sample is a tissue section.

3. The method of claim 1, wherein the biological sample is a skin sample.

4. The method of any one of claim 1 to 3, wherein the fluorophore is selected among AF532, AF488, AF546, AF594, AF647, AF700, APC-CY7, BV650, EFLUOR 660, EFLUOR 450, AND SB645.

5. The method of claim 1, wherein the fluorophores are selected among:

   1) AF-532, AF488, AF594, APC-Cy7, BV650, eFluor 660, and eFluor 450; or
   2) AF-532, *AF405,* AF488, AF647, AF546, *AF594,* AF700, and SB645.

6. The method of any one of claim 1 to 5, wherein the probe is an antibody or an antibody fragment.

7. The method of any one of claim 1 to 6, wherein the method is for identifying and characterizing immune cells among the biological sample, and wherein each probe of the first set is directed against immune cells markers and, eventually, immune cells activation markers.

8. The method of claim 7, wherein said first set of probes comprises at least one probe directed against CD45.

9. The method of any one of claim 7 or 8, wherein:

   - the first set of probes consists in probes directed against the markers CD1c, CD3, CD4, CD8, CD20, CD45, CD57, CD123, CD207, HLA-DR, MPO, Siglec8, TCR gamma/delta, and/or Tryptase; end
   - the corresponding defined biomarkers' signatures of step v) are as follows:

| Immune cell population | Biomarkers' signature |
|---|---|
| B cells | CD45$^+$ CD20$^+$ |
| NK cells | CD45$^+$ CD20$^-$ CD3$^-$ CD57$^+$ |
| CD4$^+$ T cells | CD45$^+$ CD20' CD3$^+$ TCRgd$^-$ CD4$^+$ CD8$^-$ CD57$^{low\ or\ high}$ |

(continued)

| Immune cell population | Biomarkers' signature |
|---|---|
| CD8$^+$ T cells | CD45$^+$ CD20' CD3$^+$ TCRgd$^-$ CD4$^-$ CD8$^+$ CD57$^{low\ or\ high}$ |
| gd T cells | CD45$^+$ CD20$^-$ CD3$^+$ TCRgd$^+$ |
| dn T cells | CD45$^+$ CD20$^-$ CD3$^+$ TCRgd$^-$ CD4$^-$ CD8$^-$ |
| dp T cells | CD45$^+$ CD20$^-$ CD3$^+$ TCRgd$^-$ CD4$^+$ CD8$^+$ |
| Mast cells | CD45$^+$ Tryptase$^+$ |
| DC | CD45$^+$ CD1c$^+$ CD207$^-$ HLA-DR$^{low\ or\ high}$ |
| LC | CD45$^+$ CD1c$^-$ CD207$^+$ HLA-DR$^{low\ or\ high}$ |
| DC CD207$^+$ | CD45$^+$ CD1c$^+$ CD207$^+$ HLA-DR$^{low\ or\ high}$ |
| Neutrophils | CD45$^+$ CD1c$^-$ CD207$^-$ Tryptase$^-$ Siglec8$^-$ MPO$^+$ |
| Eosinophils | CD45$^+$ CD1c$^-$ CD207$^-$ MPO$^-$ Tryptase$^-$ Siglec8$^+$ CD123$^-$ |
| Basophils | CD45$^+$ CD1c$^-$ CD207$^-$ MPO' Siglec8$^+$ CD123$^+$ |

10. The method of claim 7, wherein the immune cells are lymphoid cells, the first set of probes consists in probes directed against the markers CD3, CD4, CD8, CD20, CD45, CD57, and/or TCR gamma/delta; and the corresponding defined biomarkers' signatures of step v) are as follows:

| Immune cell population | Biomarkers' signature |
|---|---|
| B cells | CD45$^+$ CD20$^+$ |
| NK cells | CD45$^+$ CD20$^-$ CD3$^-$ CD57$^+$ |
| CD4$^+$ T cells | CD45$^+$ CD20' CD3$^+$ TCRgd$^-$ CD4$^+$ CD8' CD57$^{low\ or\ high}$ |
| CD8$^+$ T cells | CD45$^+$ CD20' CD3$^+$ TCRgd$^-$ CD4' CD8$^+$ CD57$^{low\ or\ high}$ |
| gd T cells | CD45$^+$ CD20$^-$ CD3$^+$ TCRgd$^+$ |
| dn T cells | CD45$^+$ CD20$^-$ CD3$^+$ TCRgd$^-$ CD4$^-$ CD8$^-$ |
| dp T cells | CD45$^+$ CD20$^-$ CD3$^+$ TCRgd$^-$ CD4$^+$ CD8$^+$ |

11. The method of claim 7, wherein the immune cells are myeloid cells, the first set of probes consists in probes directed against the markers CD1c, CD45, CD57, CD123, HLA-DR, MPO, Siglec8, and/or Tryptase; and the corresponding defined biomarkers' signatures of step v) are as follows:

| Immune cell population | Biomarkers' signature |
|---|---|
| Mast cells | CD45$^+$ Tryptase$^+$ |
| DC | CD45$^+$ CD1c$^+$ CD207$^-$ HLA-DR$^{low\ or\ high}$ |
| LC | CD45$^+$ CD1c$^-$ CD207$^+$ HLA-DR$^{low\ or\ high}$ |
| DC CD207$^+$ | CD45$^+$ CD1c$^+$ CD207$^+$ HLA-DR$^{low\ or\ high}$ |
| Neutrophils | CD45$^+$ CD1c$^-$ CD207$^-$ Tryptase$^-$ Siglec8$^-$ MPO$^+$ |
| Eosinophils | CD45$^+$ CD1c$^-$ CD207$^-$ MPO$^-$ Tryptase$^-$ Siglec8$^+$ CD123$^-$ |
| Basophils | CD45$^+$ CD1c$^-$ CD207$^-$ MPO' Siglec8$^+$ CD123$^+$ |

12. The method of any one of claim 1 to 6, wherein the method is for identifying, and characterizing structural cells among the biological sample, wherein each probe of the first set is directed against structural cells markers.

13. The method of claim 12, wherein the structural cells markers are selected in the group comprising ACTA2, ADIPOQ, ADIPOR1, ADIPOR2, APCDD1, ASPN, AXIN2, CALCA, CAPN3, CCL19, CDC42EP3, CIDEA, CITED1, COL1A1, COL6A1, COL6A2, COL3A1, COLEC12, COL18A1, CTHRC1, DCN, DCT, FABP4, FGF21, GLDN, GLUT4/SLC2A4, HRT15, IGFBP7, KRT14, KRTS, KRT1, KRT10, KRT6A, KRT16, KRT6b, KRT17, KRT79, LUM, MFAP4, MGP, MFAP5, MLANA, MITF , MMP2, MRGPRA, MRGPRD, NRXN1, PECAM1, PGFRA, PDGFRA PGP9.5, PHLDA1, PLIN1, PLIN2, PNPLA2, PMEL, PTGDS, QPCT, TAC1, TAGLN, TOP2A, TRPV1, TYR, TYRP1, UBEC2, UCP1, VIM, and VWF.

14. The method of claim 13, wherein the corresponding defined biomarkers' signatures of step v) are as follows:

- Keratinocytes: HRT15, KRT14, KRTS, KRT1, KRT10, KRT6A, KRT16, TOP2A, UBEC2, KRT6b, KRT17
- Melanocytes: MLANA, PMEL, TYRP1, DCT, CDC42EP3, MITF, QPCT, IGFBP7, TYR, CAPN3, PHLDA1
- Fibroblasts: COL1A1, DCN, COL6A1, COL6A2, MFAP4, COL3A1, MMP2, LUM, VIM, PDGFRA, APCDD1, AXIN2, COLEC12, PTGDS, COL18A1, MGP, MFAP5, CTHRC1, APCDD1, CCL19, ASPN, PGFRA
- Sebaceous gland cells: KRT79
- Pericytes: TAGLN, ACTA2
- Neuronal cells: NRXN1, TRPV1, MRGPRD, MRGPRA, TAC1, CALCA, B3TUB, PGP9.5
- Schawn cells: GLDN
- VEC: PECAM1, VWF
- Adipocytes: FABP4, PLIN1, PNPLA2, UCP1, CIDEA, CITED1, FGF21, ADIPOR1, ADIPOR2, GLUT4/SLC2A4, PLIN2, ADIPOQ.

Europäisches
Patentamt

European
Patent Office

Office européen
des brevets

## EUROPEAN SEARCH REPORT

Application Number

EP 22 21 2088

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/171220 A1 (AUCKLAND UNISERVICES LTD [NZ]) 2 September 2021 (2021-09-02) <br> * the whole document * <br> * abstract * <br> * page 5, line 1 – line 12 * <br> * page 5, line 13 – line 27 * <br> * figures 1-13; example 1 * <br> * claims 1-10, 20 * <br> * claims 25-28, 31-34, 44, 48-49 * <br> ----- | 1-14 | INV. <br> G01N33/533 <br> G01N21/64 <br> G01N33/50 <br> G01N33/58 |
| X | MANON SCHOLAERT ET AL: "ESDR370 – MANTIS®: an integrated digital process for 3-D deconstruction of human skin immune landscape", <br> 51ST ANNUAL ESDR MEETING, <br> 27 September 2022 (2022-09-27), – 1 October 2022 (2022-10-01), pages 1-1, <br> XP093049745, <br> DOI: 10.26226/m.62fa009edcd2c8001911eadf <br> * the whole document * <br> ----- | 1-3,7-14 | |
| X | "MANTIS® Spatial Biology Platform (website)", <br> , <br> 6 December 2021 (2021-12-06), pages 1-1, <br> XP093049750, <br> Retrieved from the Internet: <br> URL:https://genoskin.com/spatial-biology-m antis-platform/ <br> [retrieved on 2023-05-25] <br> * the whole document * <br> ----- <br> -/-- | 1-3,6-14 | TECHNICAL FIELDS SEARCHED (IPC) <br><br> G01N |

The present search report has been drawn up for all claims

| Place of search | Date of completion of the search | Examiner |
|---|---|---|
| The Hague | 26 May 2023 | Gall-Truchot, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                     
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 21 2088

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Genoskin: "Discover MANTIS® – A unique spatial biology solution", , 6 December 2021 (2021-12-06), XP093049554, Retrieved from the Internet: URL:https://www.youtube.com/watch?v=zN_KYPxY_xI [retrieved on 2023-05-25] * the whole document * | 1-14 | |
| A | HICKEY JOHN W ET AL: "Spatial mapping of protein composition and tissue organization: a primer for multiplexed antibody-based imaging", NATURE METHODS, NATURE PUBLISHING GROUP US, NEW YORK, vol. 19, no. 3, 22 November 2021 (2021-11-22), pages 284-295, XP037714874, ISSN: 1548-7091, DOI: 10.1038/S41592-021-01316-Y [retrieved on 2021-11-22] * the whole document * | 1-14 | |

TECHNICAL FIELDS
SEARCHED (IPC)

The present search report has been drawn up for all claims

| Place of search | Date of completion of the search | Examiner |
|---|---|---|
| The Hague | 26 May 2023 | Gall-Truchot, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 2088

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2023

| Patent document cited in search report | | Publication date | Patent family member(s) | | Publication date |
|---|---|---|---|---|---|
| WO 2021171220 | A1 | 02-09-2021 | AU | 2021226178 A1 | 11-08-2022 |
| | | | CA | 3170429 A1 | 02-09-2021 |
| | | | CN | 115066611 A | 16-09-2022 |
| | | | EP | 4111195 A1 | 04-01-2023 |
| | | | US | 2023095395 A1 | 30-03-2023 |
| | | | WO | 2021171220 A1 | 02-09-2021 |

EPO FORM P0459

For more details about this annex : see Official Journal of the European Patent Office, No. 12/82